# EUROPEAN PATENT APPLICATION

(11) **EP 1 479 689 A1**
(43) Date of publication of application: **24.11.2004**
(21) Application number: 03011498.7
(22) Date of filing: 21.05.2003
(51) Int. Cl.: C07K 5/00, C12N 15/00, C12N 1/21, G01N 33/00

(54) **Peptide complex**

(71) Applicant: Biotech Tools S.A., 1120 Bruxelles (BE)
(72) Inventor: Hénot, Frédéric, 1040 Bruxelles (BE); Legon, Thierry, 3360 Korbeek Lo (BE); Galy-Fauroux, Isabelle, 78670 Villennes-sur-seine (FR); Duchateau, Jean, 7060 Soignies (BE)
(74) Representative: Schreiber, Christoph

(57) **Abstract**

A complex comprising at least one heat shock protein (HSP) and at least one peptide selected from the group consisting of

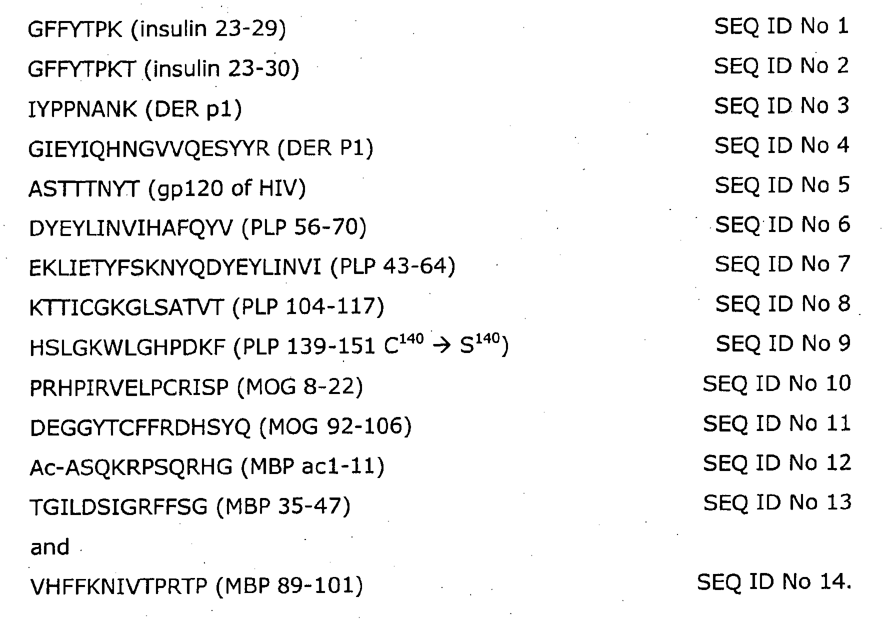

## Description

The present invention relates to complexes of heat shock proteins and tolerogenic peptides.

It is known that complexes of peptides together with heat shock proteins are suitable for inducing tolerance.

US 6,312,711 discloses a pharmaceutical or food composition intended for treating pathologies associated with graft rejection or an allergic or autoimmune reaction comprising the administration of a complex of a stress protein and epitopes of an antigenic structure.

Surprisingly, the inventors of the present invention have been able to isolate small peptides which in complex with heat shock proteins are especially useful for prevention or treatment of allergy, graft rejection or autoimmune diseases.

The inventors identified the following sequences

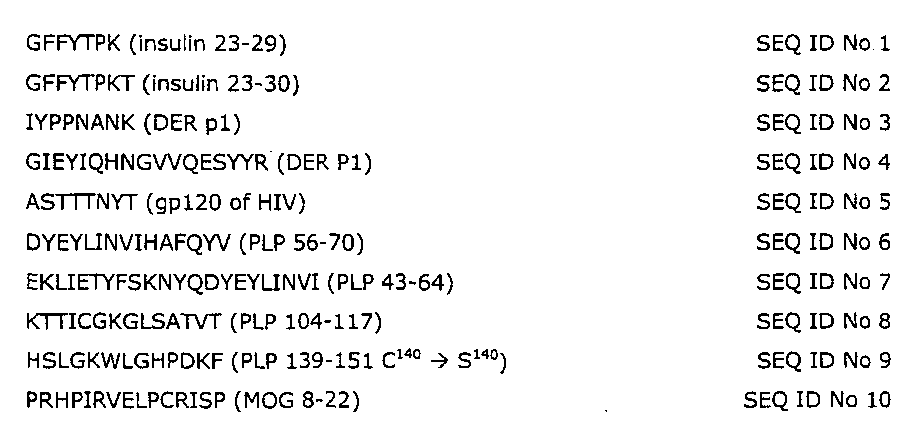

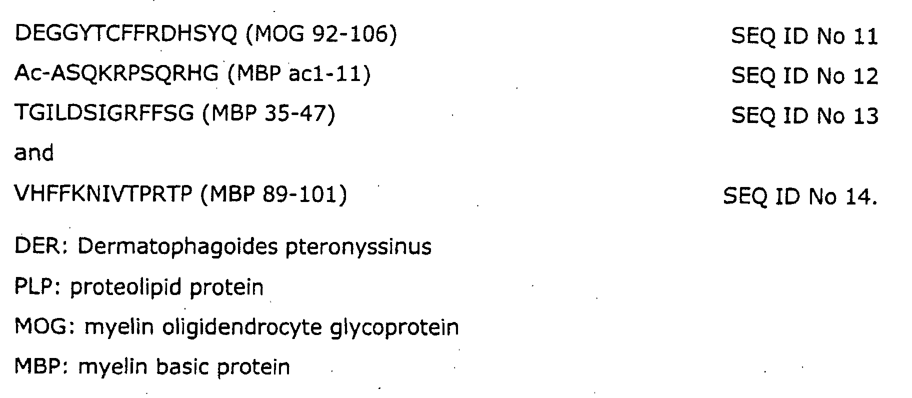

In one aspect of the invention, the invention provides a complex comprising at least one heat shock protein (HSP) and at least one peptide selected from the group consisting of

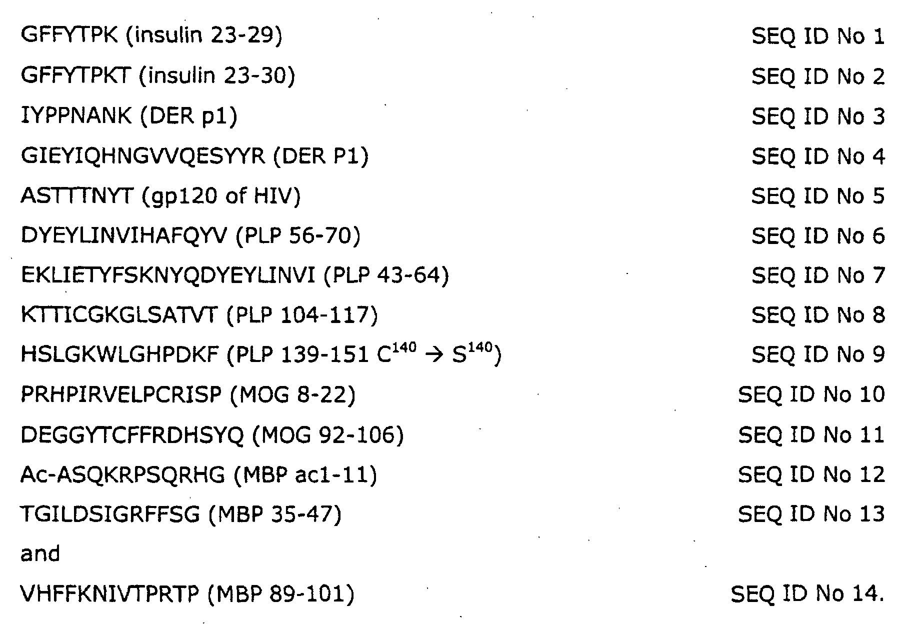

In a preferred embodiment, the HSP is a bacterial HSP and more preferably a bacterial HSP from a saprophytic bacteria. Suitable HSP can be selected from hsp40, hsp70, grpE, hsp90, CPN60 (hsp60), FK506-binding proteins, gp96, calrecticulin, hsp110, grp170 and hsp100, alone or in combination.

According to the invention either complete HSP or fragments of the HSP can be used. A preferred fragment is the polypeptide binding domain of the heat shock protein.

Another aspect of the invention is a nucleic acid encoding at least one of the peptides having Seq. ID Nos. 1 to 14.

In a further aspect, the invention provides a nucleic acid encoding at least one HSP and at least one peptide comprising at least one of the sequences selected from Seq. ID No. 1 to Seq. ID No. 14. This nucleic acid can be used to express the corresponding peptide in vitro or in a cell or in a patient. For expression in a patient, a gene delivery vehicle would be necessary. Therefore, the gene delivery vehicle comprising at least the nucleic acid of the invention is also part of the invention.

Such a gene delivery vehicle would transfect a cell. Therefore, a cell transfected by at least one gene delivery vehicle of the invention is also part of the invention.

Either the complex or the gene delivery vehicle could be administered to patient in need thereof. Therefore, a pharmaceutical or food composition comprising the complex or the gene delivery vehicle of the present invention is also an aspect of the invention.

The complex of the present invention can be prepared by hydrolyzing *in-vitro* at least one protein with at least one protease to obtain hydrolyzed fragments and combing *in-vitro* the hydrolyzed fragments with at least one heat shock protein to obtain at least one complex.

Alternatively, the complex of the present invention can be prepared by synthesizing the peptides identified as Seq. ID No. 1 to Seq. ID No. 14 and combining the synthesized peptide with at least one heat shock protein. Suitable procedures are described by Merrifield, 1963, J. Am. Chem. Soc. 85:2149.

In a preferred embodiment, the peptide-HSP complex is formed under reducing conditions in a buffer containing at least one reducing agent. Suitable reducing agents are for example dithiothreitol and beta-mercaptoethanol. The most preferred concentrations of the reducing agent are from 0.001 mM to 700 mM, and more preferably between 0.1 mM and 50 mM.

In another embodiment, the peptide-HSP complex is formed under oxidative conditions in a buffer containing at least one oxidative agent. A suitable oxidative agent is for example hydrogen peroxide. The most preferred concentrations of the oxidative agent are from 0.1 mM to 100 mM, and more preferably between 0.5 mN and 20mN.

A further aspect of the invention is to use the complex of the present invention as a carrier for the delivery of biologically active molecules. The peptides can be linked to biologically active molecules e.g. nucleic acids, peptides, polypeptides, proteins, polysaccharides, lipids, drugs, and can then be combined with the heat shock protein.

In a further aspect the invention provides the isolated peptide selected from the group consisting of

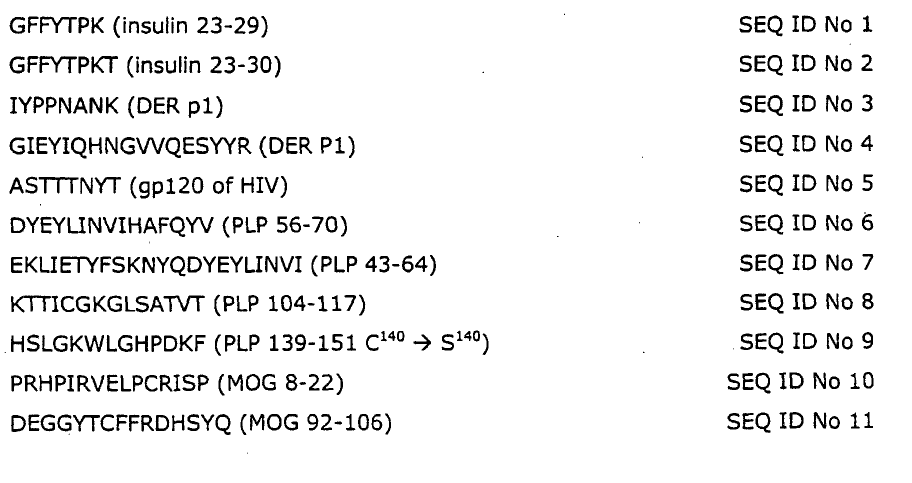

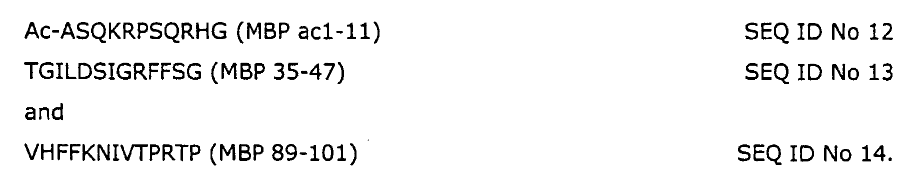

Also a complex of these peptides with at least one other protein such as but not limited to antibodies is part of the invention.

The complex of the present invention or the gene delivery vehicle of the invention are useful for the prevention or treatment of allergy, graft rejection or autoimmune diseases.

The complex of the present invention can also be used for the induction of a cellular reponse comprising cells able to secrete immunosuppressive cytokines after exposure to at least one antigen comprising at least one sequence selected from SEQ ID No 1 to SEQ ID No 14.

A further aspect of the invention is a diagnostic composition comprising the complex of the invention.

A further aspect of the invention is a method for the in vitro diagnosis of a disease comprising the steps of
- bringing a biological fluid of an animal containing proteins into contact with the diagnostic composition of the invention
- measuring binding of said proteins with said diagnostic composition
wherein binding indicates a disease of the animal.

Another aspect of the invention is to provide with antibodies specific for at least one of the peptides of the inventions or specific for at least one complex of the present invention. The antibody can be a polyclonal antibody, a monoclonal antibody or fragments of such as but not limited to Fab and F(ab')₂.

The antibody of the present invention can be used for the prevention or treatment of allergy, graft rejection or autoimmune disease. Also the antibody of the present invention can be used in a method for the in vitro diagnosis of a disease such as allergy, graft rejection or autoimmune disease.

### Examples

### Protein preparation

Before enzymatic digestions, the proteins of interest are extensively washed with water by centrifugation through a centricon YM-10 assembly to remove any low molecular weight material loosely associated with it.

### Trypsin digestion

One to five milligrams of the protein of interest is dissolved in 1 to 5 mL of phosphate buffer 40 mM pH 8.0 (final concentration of 1 mg/mL). The solution is incubated for 10 minutes at 100°C. The solution is then rapidly cooled at 4°C, and 18 to 90 µL of β-mercaptoethanol is added (1.8% v/v). The resulting solution is incubated at 37°C for 10 minutes and 20 to 100 µL of trypsin solution (10 mg/mL of Tris•HCl 40 mM pH 8.0 final ratio protein/protease of 0.1) is added to the protein solution.

The resulting solution is incubated at 37°C for six hours. The solution is then centrifuged through a centricon YM-10 assembly to remove the remaining protein and trypsin.

### DnaK.ATP complex preparation

25 µL of ATP solution (4.5 mg/mL of buffer 1 (25 mM HEPES, 10 mM KCI, 3 mM magnesium chloride, 5 mM beta-mercaptoethanol, pH 7.5) is added to 400 µL of DnaK (2 mg/mL of buffer 1). The solution is incubated at 20°C for one hour, and then centrifuged through a centricon YM-10 assembly to remove any low molecular weight material loosely associated with Dna K. The large molecular weight fraction is removed, and washed extensively with buffer 1 by ultrafiltration using a centricon YM-10.

### In vitro production of stress protein-peptide complexes. Isolation of the bound peptides

The ultrafiltrated and neutralized pepsin or trypsin digestion is mixed with the ATP-pre-treated DnaK to give at least a 1:1 (w:w) DnaK:peptides ratio. Then, ADP is added (1 mM final) and the mixture is incubated for at least one hour at 25°C in the suitable buffer 1. The preparations are centrifuged through a centricon YM-10 assembly to remove the remaining unbound peptides. The low and the large molecular weight fractions are recovered. The large molecular weight fraction containing DnaK-peptide complexes is washed extensively with buffer 1 containing 1 mM ADP by ultrafiltration using a centricon YM-10. The bound peptides are eluted by incubating the HSP-peptide complexes in a low pH buffer. A last ultrafiltration using a centricon YM-10 is removing the large molecular weight fraction.

### HPLC analysis

The resulting low molecular weight fractions are fractionated by reverse phase high pressure liquid chromatography (HPLC) using a Vydac C18 reverse phase column (HP32, 201TP52 C18, 250/2.1 mm, 5 µm (available from Vydac)). The elution of the peptides can be monitored at both OD 214 nm and OD 280 nm.

### Biological studies

Animals: Adult 6-8 week ABH mice will be injected subcutaneously in the flank with 1 mg of freeze dried mouse spinal cord homogenate (SCH) in Freund's adjuvant on day 0 & 7.

This mouse model is an experimental allergic encephalomyelitis (EAE) mouse model of multiple sclerosis (MS) described in Baker et al. (1990). J. Neuroimmunology 28:261.

Testing of the products will be made in blind. Solutions for administration to the mice will be encoded. The correspondence between the codes and the vials contains will be given to an independent expert.

### I.1 Treatment of mice

### I.1.1. Experimental group sizes

4 groups of 8-10 mice to be treated.

### I.1.2. Active molecules and doses to test

For the peptide group (group 1): 10.0 µg of peptide PLP 56-70 per mouse (Total dose)
For the adjuvant group (group 2): 10.0 µm of E.coli Heat Shock Proteins per mouse (Total dose)
For the peptide + adjuvant group (group 3): A vaccine containing 10.0 µg of peptide PLP 56-70 mixed with 10,0 µg of E.coli Heat Shock Proteins per mouse (Total dose)
For the peptide + adjuvant group (group 4): A vaccine containing 1.0 µg of peptide PLP 56-70 mixed with 1.0 µg of E.coli Heat Shock Proteins per mouse (Total dose)

### I.1.3. Route of administration

One route, oral administration is tested by buccal injection in micro-dose of 0.010 mL.

### I.1.4 Dosage

Vaccine is administered in 5 equivalent doses (total dose divided into 5) every two days from the first day of the treatment D-9 (start treatment 9 days before the first sensitisation D 0).

Mice are individually treated.

### I.2 Clinical outcomes

### I.2.1. Follow-up of the clinical disease

Clinical disease will be assessed by a blinded observer and rated on a clinical scale on the all treated animals.

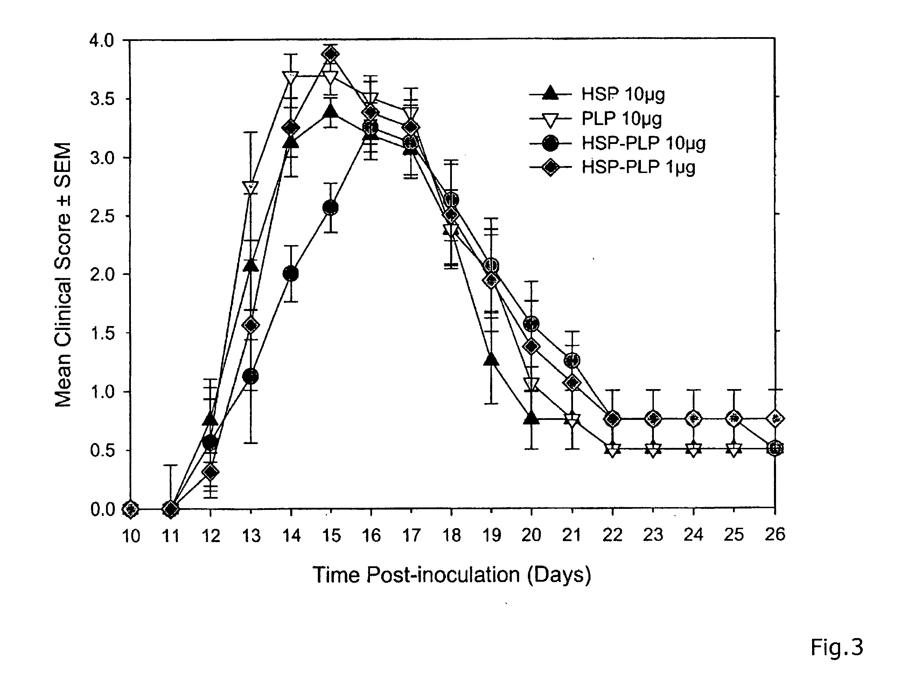

RESULT: There is a diminution of severity in group 3-treated animals as they did not appear to stay paralysed for as long. This is evident when severity of sign are plotted on a daily basis as shown Figure 3. This demonstrates an adjuvant effect of DnaK.

### Figures

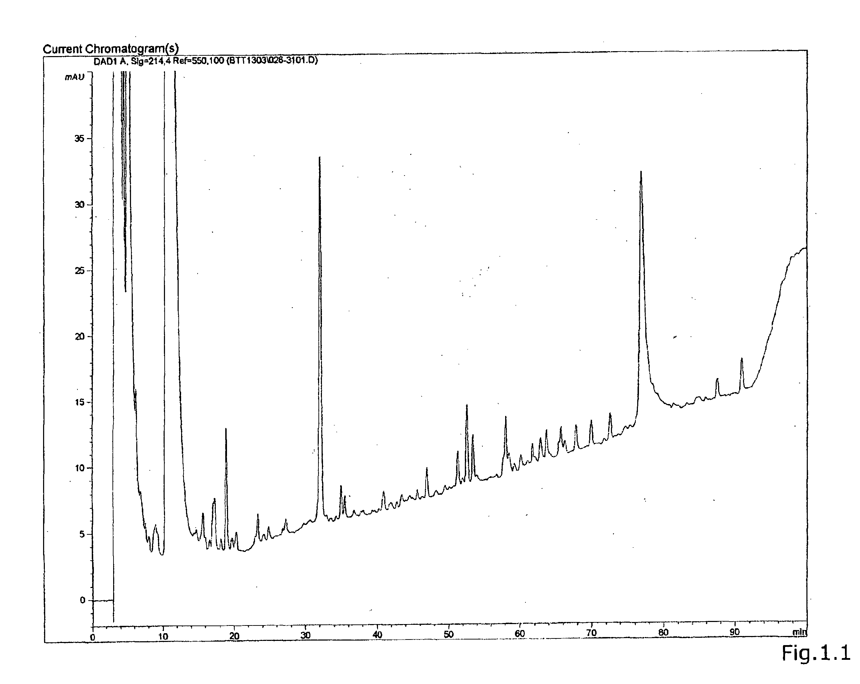

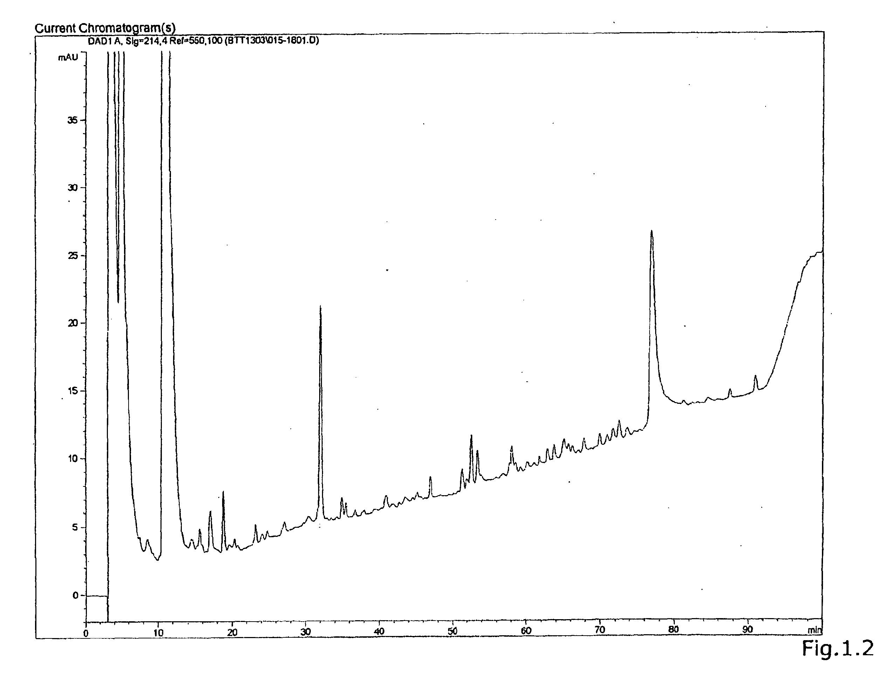

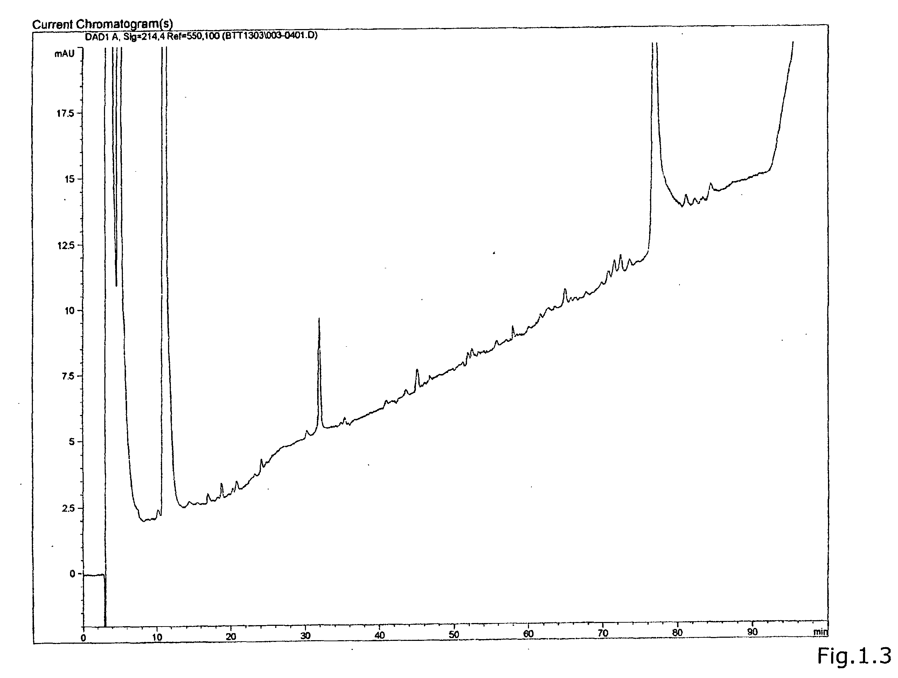

Figure 1.1: peptides (MW < or = 10 kDa) generated by trypsin-cleavage of Derp1.
Figure 1.2: unbond peptides (MW < or 10 kDa) to DnaK, from the trypsin-cleavage of Derp1.
Figure 1.3: peptides from the trypsin-cleavage of Derp1 (MW < or = 10 kDa) that were bound to DnaK.

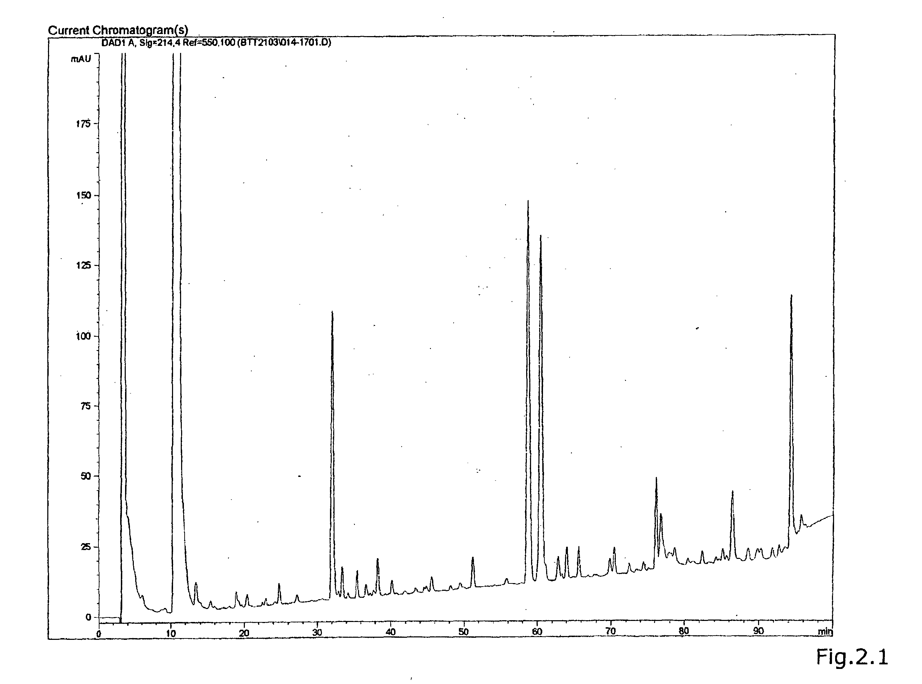

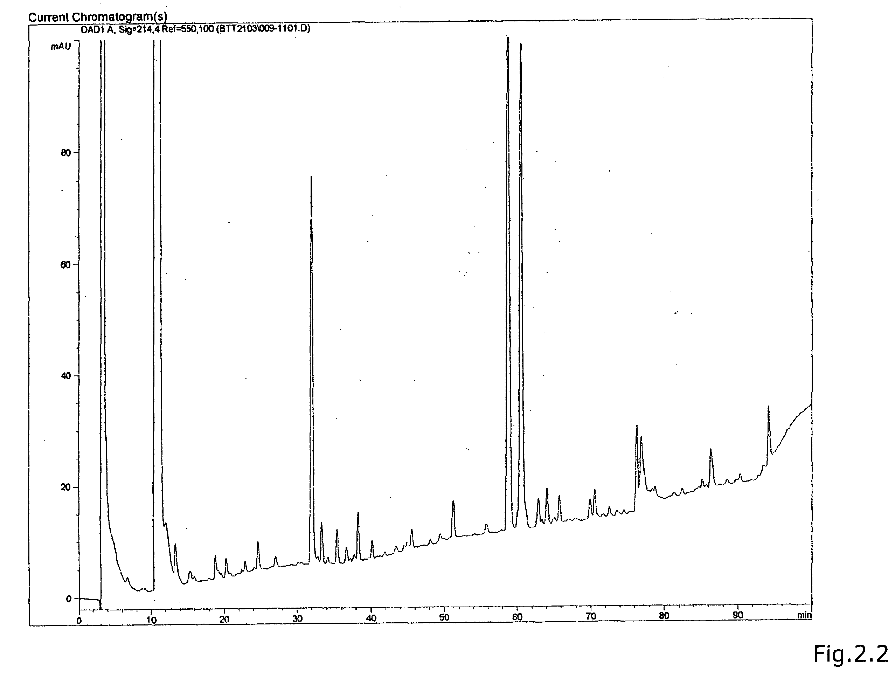

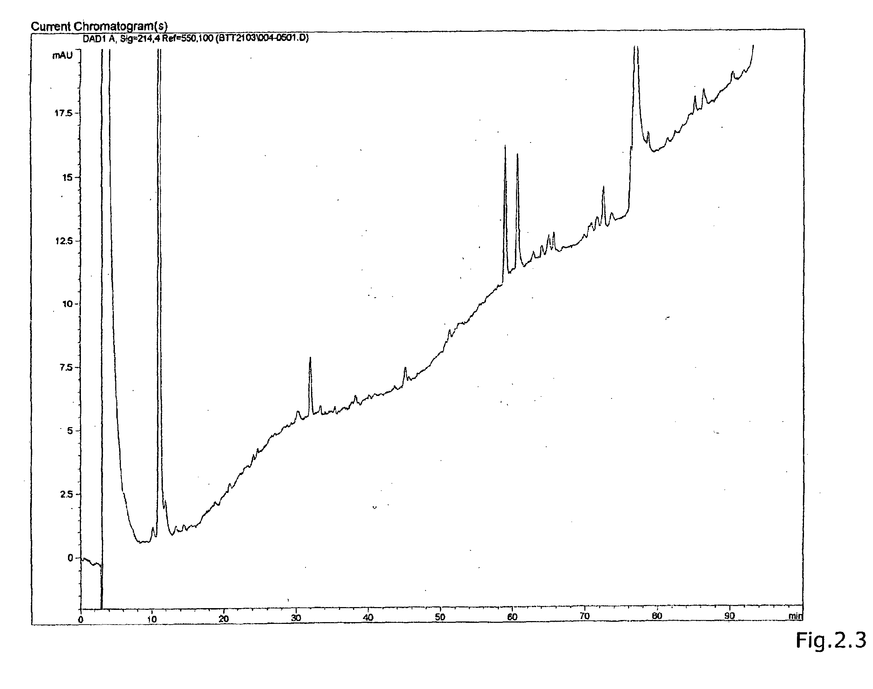

Figure 2.1: peptides (MW < or 10 kDa) generated by trypsin-cleavage of insulin.
Figure 2.2: unbound peptides (MW < or 10 kDa) to DnaK, from the trypsin-cleavage of insulin.
Figure 2.3: peptides from the trypsin-cleavage of insulin (MW < or = 10 kDa) that were bound to DnaK.
Figure 3: Mean clinical score in function of time post-inoculation

## Claims

1. A complex comprising at least one heat shock protein (HSP) and at least one peptide selected from the group consisting of

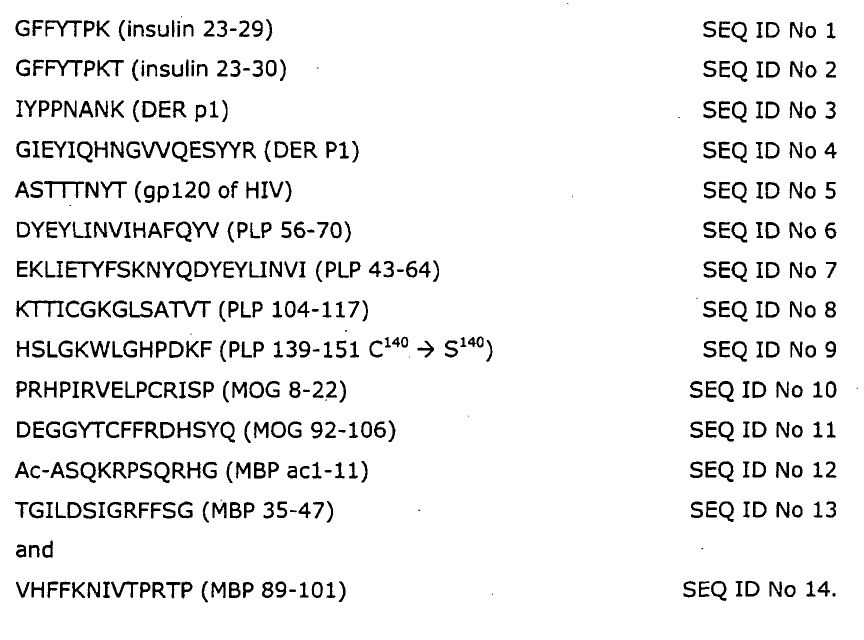

2. The complex according to claim 1, wherein the HSP is a bacterial HSP.

3. The complex according to of claim 1 or 2, wherein the HSP is selected from hsp40, hsp70, grpE, hsp90, CPN60 (hsp60), FK506-binding proteins, gp96, calrecticulin, hsp110, grp170 and hsp100.

4. The complex according to any of claims 1 to 3, wherein the heat shock protein is the polypeptide binding domain of a heat shock protein.

5. A nucleic acid encoding at least one HSP and at least one peptide comprising at least one of the sequences selected from SEQ ID No 1 to SEQ ID No 14.

6. A gene delivery vehicle comprising at least one nucleic acid according to claim 5.

7. A cell transfected by at least one gene delivery vehicle according to claim 6.

8. A pharmaceutical or food compositions comprising the complex of any of claims 1 to 4 or a gene delivery vehicle of claim 6.

9. A method for preparing a complex according to any one of claims 1 to 4 comprising the steps of
• hydrolyzing *in-vitro* at least one protein with a protease to obtain hydrolyzed fragments
• combing *in-vitro* the hydrolyzed fragments with at least one heat shock protein to obtain a complex.

10. The method of claim 9, wherein unbound hydrolyzed fragments are separated from the complex.

11. A peptide selected from the group consisting of

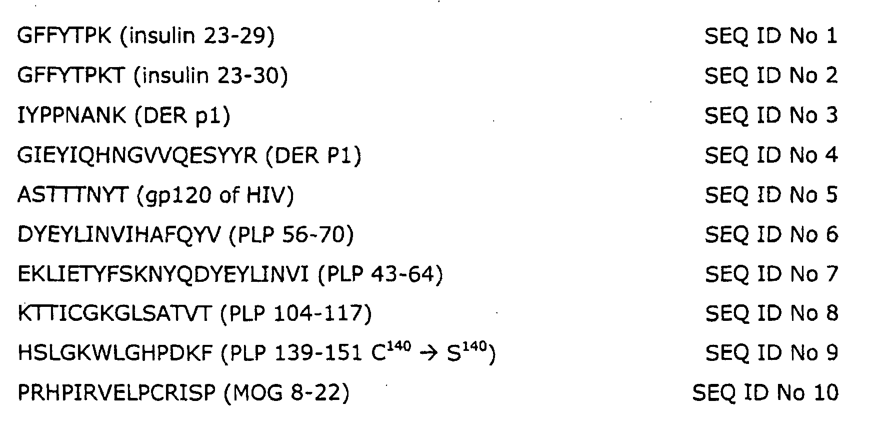

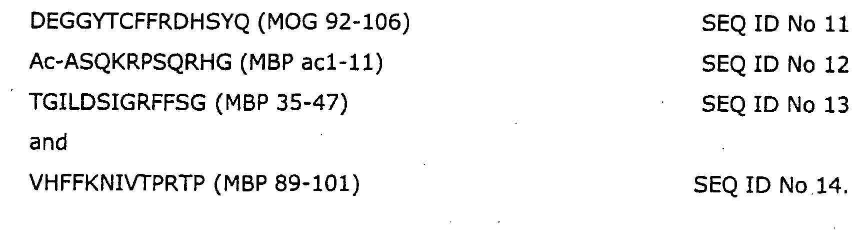

12. A complex of at least one of the peptides of claims 11 with at least one protein.

13. Use of the complex of any of claims 1 to 4 or the gene delivery vehicle of claim 6 for the prevention or treatment of allergy, graft rejection or autoimmune disease.

14. Use of the complex of any of claims 1 to 4 for the induction of a cellular response comprising cells able to secrete immunosuppressive cytokines after exposure to at least one antigen comprising at least one sequence selected from SEQ ID No 1 to SEQ ID No 14.

15. Diagnostic composition comprising the complex of any of claims 1 to 4.

16. A method for the in-vitro diagnosis of a disease comprising the steps of
• bringing a biological fluid of an animal containing proteins into contact with the diagnostic composition of claim 15
• measuring binding of said proteins with said diagnostic composition
wherein binding indicates a disease of the animal.


SEQUENCE LISTING

<110> BioTech Tools S.A.

<120> Peptide complex

<130> 022616epCS/BM

<140> 03011498.7
<141> 2003-05-21

<160> 14

<170> PatentIn Ver. 2.1

<210> 1
<211> 7
<212> PRT
<213> Artificial Sequence

<220>
<223> Description of Artificial Sequence: peptide (
      insulin 23-29)

<400> 1
Gly Phe Phe Tyr Thr Pro Lys
  1               5


<210> 2
<211> 8
<212> PRT
<213> Artificial Sequence

<220>
<223> Description of Artificial Sequence: peptide
      (insulin 23-30)

<400> 2
Gly Phe Phe Tyr Thr Pro Lys Thr
  1               5


<210> 3
<211> 8
<212> PRT
<213> Artificial Sequence

<220>
<223> Description of Artificial Sequence: peptide (DER
      p1)

<400> 3
Ile Tyr Pro Pro Asn Ala Asn Lys
  1               5


<210> 4
<211> 17
<212> PRT
<213> Artificial Sequence

<220>
<223> Description of Artificial Sequence: peptide (DER
      P1)

<400> 4
Gly Ile Glu Tyr Ile Gln His Asn Gly Val Val Gln Glu Ser Tyr Tyr
  1               5                  10                  15

Arg



<210> 5
<211> 8
<212> PRT
<213> Artificial Sequence

<220>
<223> Description of Artificial Sequence: peptide (gp120
      of HIV)

<400> 5
Ala Ser Thr Thr Thr Asn Tyr Thr
  1               5


<210> 6
<211> 15
<212> PRT
<213> Artificial Sequence

<220>
<223> Description of Artificial Sequence: peptide (PLP
      56-70)

<400> 6
Asp Tyr Glu Tyr Leu Ile Asn Val Ile His Ala Phe Gln Tyr Val
  1               5                  10                  15


<210> 7
<211> 22
<212> PRT
<213> Artificial Sequence

<220>
<223> Description of Artificial Sequence: peptide (PLP
      43- 64)

<400> 7
Glu Lys Leu Ile Glu Thr Tyr Phe Ser Lys Asn Tyr Gln Asp Tyr Glu
  1               5                  10                  15

Tyr Leu Ile Asn Val Ile
             20


<210> 8
<211> 14
<212> PRT
<213> Artificial Sequence

<220>
<223> Description of Artificial Sequence: peptide (PLP
      104-117)

<400> 8
Lys Thr Thr Ile Cys Gly Lys Gly Leu Ser Ala Thr Val Thr
  1               5                   10

<210> 9
<211> 13
<212> PRT
<213> Artificial Sequence

<220>
<223> Description of Artificial Sequence: peptide (PLP
      139-151 C140 > S140)

<400> 9
His Ser Leu Gly Lys Trp Leu Gly His Pro Asp Lys Phe
  1               5                   10

<210> 10
<211> 15
<212> PRT
<213> Artificial Sequence

<220>
<223> Description of Artificial Sequence: peptide (MOG
      8-22)

<400> 10
Pro Arg His Pro Ile Arg Val Glu Leu Pro Cys Arg Ile Ser Pro
  1               5                   10                  15

<210> 11
<211> 15
<212> PRT
<213> Artificial Sequence

<220>
<223> Description of Artificial Sequence: peptide (MOG
      92-106)

<400> 11
Asp Glu Gly Gly Tyr Thr Cys Phe Phe Arg Asp His Ser Tyr Gln
  1               5                   10                  15

<210> 12
<211> 11
<212> PRT
<213> Artificial Sequence

<220>
<223> Description of Artificial Sequence: peptide (MBP
      ac1-11)

<400> 12
Ala Ser Gln Lys Arg Pro Ser Gln Arg His Gly

1                 5                   10


<210> 13
<211> 13
<212> PRT
<213> Artificial Sequence

<220>
<223> Description of Artificial Sequence: peptide (MBP
      35-47)

<400> 13
Thr Gly Ile Leu Asp Ser Ile Gly Arg Phe Phe Ser Gly
  1               5                   10


<210> 14
<211> 13
<212> PRT
<213> Artificial Sequence

<220>
<223> Description of Artificial Sequence: peptide (MBP
      89-101)

<400> 14
Val His Phe Phe Lys Asn Ile Val Thr Pro Arg Thr Pro
  1               5                   10